# EUROPÄISCHE PATENTSCHRIFT

(11) **EP 1 221 992 B1**
(45) Veröffentlichungstag und Bekanntmachung des Hinweises auf die Patenterteilung: **16.01.2008**
(21) Anmeldenummer: 00958087.9
(22) Anmeldetag: 19.09.2000
(51) Int. Cl.: A61M 5/168

(54) **VORRICHTUNG ZUR DOSIERTEN VERABREICHUNG EINES INJIZIERBAREN PRODUKTS**
DEVICE FOR THE CONTROLLED ADMINISTRATION OF AN INJECTABLE PRODUCT
DISPOSITIF POUR ADMINISTRER UNE DOSE DE PRODUIT INJECTABLE

(30) Priorität: 05.10.1999 DE 19947826
(43) Veröffentlichungstag der Anmeldung: 17.07.2002
(73) Patentinhaber: Disetronic Licensing AG, 3401 Burgdorf (CH)
(72) Erfinder: KIPFER, Urs, CH-3432 Lützelflüh (CH)
(74) Vertreter: Schalch, Rainer
(86) Internationale Anmeldenummer: PCT/CH2000/000506
(87) Internationale Veröffentlichungsnummer: WO 2001/024854

(56) Entgegenhaltungen:
- EP-A- 0 778 001
- WO-A-00/10628
- WO-A-96/27398
- WO-A-99/15214
- US-A- 4 529 401
- US-A- 5 764 159

## Beschreibung

Die Erfindung betrifft eine Vorrichtung zur dosierten Verabreichung eines injizierbaren Produkts.

Infusionsgeräte, wie sie beispielsweise in der Diabetestherapie eingesetzt werden und wie die Erfindung sie insbesondere auch betrifft, weisen zumindest folgende Komponenten auf: ein Gehäuse, ein von dem Gehäuse aufgenommenes Behältnis mit dem Produkt, ein Fördermittel zur Förderung des Produkts aus dem Behältnis und eine Antriebseinrichtung für das Fördermittel. Das Produkt wird aus dem Behältnis durch einen Katheter und eine daran angeschlossene Infusionsnadel hindurch subkutan verabreicht.

Benutzer, die sich das Produkt im Rahmen einer Therapie oder zu sonstigen Zwecken selbst verabreichen, legen im allgemeinen großen Wert auf eine diskrete bzw, unauffällige Benutzbarkeit ihres Infusionsgeräts. Die Sicherheit in der Handhabung des Geräts soll dabei jedoch nicht leiden. Ferner trägt auch eine bequeme und einfache Handhabbarkeit des Geräts zur Sicherheit in der Verabreichung des Produkts bei.

Eine Vorrichtung zur dosierten Verabreichung eines injizierbaren Produkts gemäss dem Oberbegriff des Anspruch 1 ist aus dem Document WO-A-96 27398 bekannt.

Aufgabe der Erfindung ist es, eine Vorrichtung zur dosierten Verabreichung eines injizierbaren Produkts zu schaffen, die die keibungskräfte und die aufgebrachte kraft unverfälscht auf den kraftvensor übertragen wird.

Diese Aufgabe wird durch den Gegenstand des unabhängigen Anspruch 1 gelöst.

Die Erfindung geht von einer Vorrichtung zur dosierten Verabreichung eines injizierbaren Produkts aus, die wenigstens die folgenden Komponenten umfasst: ein Gehäuse, ein von dem Gehäuse aufgenommenes Behältnis für das Produkt, ein Fördermittel zur Förderung des Produkts aus dem Behältnis und eine Antriebseinrichtung für das Fördermittel.

Die Antriebseinrichtung umfasst vorzugsweise einen Antriebsmotor. In einem bevorzugten Ausführungsbeispiel umfasst sie ferner einen auf das Fördermittel wirkenden Spindeltrieb. An einen Auslass des Behältnisses ist ein Katheter mit einer Injektions- oder Infusionsnadel anschließbar oder bereits angeschlossen. Das Behältnis ist vorzugsweise eine mit dem Produkt gefüllte Ampulle, die in ein von dem Gehäuse gebildetes Aufnahmefach einsetzbar ist und ausgetauscht werden kann. Wie dies beispielsweise bei bekannten Infusionspumpen in der Diabetestherapie der Fall ist, sind die vorgenannten Komponenten vorzugsweise alle in dem Gehäuse aufgenommen. Dies ist jedoch nicht unumgänglich für die Zwecke der Erfindung erforderlich. Das Produkt ist vorzugsweise eine medizinische und/oder kosmetische Wirkstofflösung, beispielsweise Insulin. Das Fördermittel ist vorzugsweise ein Kolben, kann aber auch eine Peristaltikpumpe oder anders geeignet ausgebildet sein. Die Vorrichtung wird vom Benutzer vorzugsweise ständig am Körper oder an der Kleidung getragen.

Die Vorrichtung weist eine Einrichtung zur Feststellung einer Fehlfunktion der Vorrichtung auf. Solch eine Fehlfunktion kann in einer Occlusion oder einem Leck auf dem Weg des Produkts von dem Behältnis bis zu einem Auslass der Injektionsnadel liegen. Ein weiteres Beispiel einer Fehlfunktion kann in der Antriebseinrichtung begründet sein, beispielsweise einem defekten oder gestörten Antriebsmotor für das Fördermittel oder einer defekten oder gestörten Steuerung und/oder Regelung für einen Antriebsmotor. Obgleich die Antriebseinrichtung vorzugsweise einen Antriebsmotor und eine Steuerung und/oder Regelung für den Antriebsmotor umfasst, ist auch ein nicht motorischer Antrieb des Fördermittels denkbar, beispielsweise mittels eines Druckmediums oder einer Antriebsfeder.

Vorzugsweise ist die Vorrichtung mit einer Eingaberichtung ausgestattet, die beispielsweise durch eine Tastatur oder ein Touchscreen gebildet wird. Ein anderes Eingabemittel kann grundsätzlich jedoch stattdessen oder zusätzlich dazu vorgesehen sein, beispielsweise eine Spracheeingabe. Mittels der Eingabeeinrichtung kann insbesondere der Benutzer selbst Einfluss auf die Verabreichung des Produkts nehmen.

Nach der Erfindung ist von dem Gehäuse ein Vibratormotor aufgenommen. Besonders bevorzugt ist der Vibratormotor in dem Gehäuse aufgenommen. Grundsätzlich kann er jedoch auch an dem Gehäuse außen befestigt sein. Die Anordnung des Vibratormotors erfolgt in beiden Fällen derart, dass eine vibratorische Bewegung des Vibratormotors von dem Benutzer deutlich spürbar wahrgenommen werden kann. Vorzugsweise verursacht der Vibratormotor eine Vibration des gesamten Gehäuses oder eines Teils des Gehäuses.

In einer ersten bevorzugten Funktion bildet der Vibratormotor eine Alternative zu einem akustischen oder sonstigen Signalgeber oder, was besonders bevorzugt wird, ein zusätzliches Mittel für eine Alarmierung. Wenn durch eine Einrichtung zur Feststellung einer Fehlfunktion der Vorrichtung die betreffende Fehlfunktion festgestellt wird, gibt der Vibratormotor ein vibratorisches Alarmsignal ab. In diesem Fall ist eine Ansteuerung für den Vibratormotor mit der Einrichtung zur Feststellung der Fehlfunktion verbunden. Bei Auftreten und Feststellen der betreffenden Fehlfunktion wird der Vibratormotor angesteuert und erzeugt das vibratorisches Alarmsignal.

Ein vibratorisches Alarmsignal wird von dem Benutzer selbst sicher wahrgenommen, nicht jedoch von seiner Umgebung. Mittels des Vibratormotors wird somit eine sichere und gleichzeitig diskrete, d.h. unauffällige Alarmierung des Benutzers bereitgestellt. Ist der Vibratormotor zusätzlich zu einem anderen Alarmierungsmittel, beispielsweise einem akustischen Alarmierungsmittel, vorgesehen, so wird durch den Vibratormotor eine Redundanz in Bezug auf die Alarmierungsmittel geschaffen. Ist zusätzlich ein weiteres Alarmierungsmittel vorhanden, beispielsweise ein akustischer Signalgeber, so ist vorzugsweise ein Schalter vorgesehen, mit dem der Benutzer wahlweise eines der Alarmierungsmittel ausschalten bzw. als Alarmierungsmittel auswählen kann. Vorteilhafterweise kann eine gemeinsame Ansteuerung für redundant mehrerer Alarmierungsmittel vorgesehen sein. Es entfällt dadurch zusätzlicher Aufwand zur Ansteuerung des Vibratormotors.

Die Einrichtung zur Feststellung einer Fehlfunktion weist einen Sensor auf, mit dem eine unzulässig große Abweichung von einem Idealzustand detektiert wird. Der Sensor ist vorzugsweise Bestandteil einer Einrichtung zur Überwachung eines Drucks innerhalb der Komponenten der Vorrichtung, die das Produkt führen, falls die festzustellende Fehlfunktion eine Occlusion ist. Der Sensor hierfür ist ein Kraftsensor. In solch einem Falle wird eine für den Antrieb des Fördermittels erforderliche Kraft gemessen. Vorzugsweise wird die von dem Fördermittel ausgeübte Reaktionskraft direkt gemessen. Auch zur Feststellung eines Lecks wird vorzugsweise ein Kraftsensor verwendet. Vorteilhafterweise wird mit dem gleichen Kraftsensor sowohl eine Occlusion als auch ein Leck detektiert.

Eine Fehlfunktion eines Antriebsmotors und/oder eine Fehlfunktion einer Steuerung und/oder Regelung für einen Antriebsmotor wird bevorzugt mittels eines Drehwinkellagesensors für den Motor detektiert. Besonders bevorzugt wird die von dem Sensor detektierte Drehwinkellage der Steuerung für den Antriebsmotor nicht als Regelgröße aufgegeben, sondern lediglich zum Zwecke der Feststellung der Fehlfunktion der Antriebseinrichtung und Ansteuerung des Vibratormotors verwendet.

Die Fehlfunktion kann auch in einem sich ankündigenden Aufbrauchen der gespeicherten Energie einer Energiequelle für die Antriebseinrichtung bestehen. Wird für den Antriebsmotor elektrische Energie benötigt, so kann beispielsweise der Ladezustand einer Batterie für den Antriebsmotor überwacht werden. Bei Unterschreiten einer vorgegebenen Mindestrestenergie wird in diesem Fall der Vibratormotor über die Ansteuerung angesteuert.

Falls die Vorrichtung über eine Eingabevorrichtung verfügt, besteht nach der Erfindung eine zweite Funktion des Vibratormotors in einer Bestätigung von Eingaben, die mittels der Eingabeeinrichtung eingegeben worden sind. Vorzugsweise sind ausgewählten oder sämtlichen Eingabemitteln der Eingabeeinrichtung individuelle vibratorische Signale des Vibratormotors zugeordnet. Gibt der Benutzer beispielsweise einen Sonderbolus ein, so werden die einzelnen Eingaben, die beispielsweise über eine Tastatur oder einen Touchscreen erfolgen, individuell vibratorisch bestätigt. In dieser Funktion des Vibratormotors ist eine Ansteuerung für den Vibratormotor mit der Eingabeeinrichtung verbunden.

Weist die Antriebseinrichtung eine Steuerung und/oder Regelung für einen Antriebsmotor auf, so wird die Ansteuerung für den Vibratormotor vorzugsweise durch diese Steuerung und/oder Regelung gebildet.

Ein Antriebsmotor für das Fördermittel kann gleichzeitig auch den Vibratormotor bilden. In einem weiteren bevorzugten anderen Ausführungsbeispiel ist zusätzlich zu einem Antriebsmotor für das Fördermittel ein separater Vibratormotor für eine oder eine Kombination der genannten Funktionen vorgesehen.

Eine dritte Funktion des Vibratormotors besteht nach der Erfindung darin, dass der Vibratormotor bei einem Primen der Vorrichtung vibriert. Hierdurch wird eine besonders bequeme und sichere Entlüftung des Produktführungssystems von dem Behältnis bis zu dem Auslass der Injektions- oder Infusionsnadel gewährleistet. Insbesondere muss der Benutzer bei einem Primen die Vorrichtung nicht rütteln oder an die Vorrichtung klopfen, um etwaige Luftbläschen bei dem Primen sicher zu entfernen. Das Primen ist beendet, wean das Produkt an der Injektions- oder Infusionsnadel austritt. Es dient dem Entlüften der produktführenden Komponenten.

In einer vierten Funktion wird der Vibratormotor erfindungsgemäß periodisch angesteuer:, um dem Benutzer das ordnungsgemäße Funktionieren seiner Vorrichtung taktil anzuzeigen. Dies kann insbesondere zusätzlich zu einer Alarmierungsfunktion oder auch alternativ dazu vorgesehen sein. Durch die positive Anzeige, dass die Vorrichtung ordnungsgemäß funktioniert, wird eine besonders sichere Handhabung gewährleistet.

Der erfindungsgemäß vorgesehene Vibratormotor kann nur eine einzige der vorstehend genannten vier Funktionen oder eine Kombination erfüllen. Vorzugsweise erfüllt er mehrere und besonders bevorzugt sämtliche der vier vorgenannten Funktionen.

Falls der Vibratormotor mehrere der genannten Funktionen erfüllt, kann für jede der Funktionen eine charakteristische Vibrationsbewegung erzeugt werden. Falls durch den Vibratormotor auf mehrere unterschiedliche Fehlfunktionen aufmerksam gemacht werden kann, ist vorteilhafterweise auch für jede Fehlfunktion ein charakteristisches Vibrationssignal vorgesehen. Das Vibrationssignal einer Fehlfunktion unterscheidet sich insbesondere von einem periodisch erzeugten Signal zur Bestätigung des ordnungsgemäßen Betriebs. Andererseits können jedoch Bestätigungssignale für Eingaben mit Alarmierungssignalen übereinstimmen. Auch für das Primen muss eine individuelle Vibrationsbewegung nicht vorgesehen sein.

Der Vibratormotor weist vorteilhafterweise eine größte Länge von höchstens 20 mm und eine größte Breite von höchstens 5 mm auf.

Ein bevorzugtes Ausführungsbeispiel der Erfindung wird nachfolgend anhand von Figuren erläutert. Es zeigen:
- Fig. 1: einen Längsschnitt einer Infusionspumpe mit Drucküberwachung und Vibratormotor ,
- Fig. 2: einen Kraftsensor für die Pumpe nach Figur 1 und
- Fig. 3: ein Schemaschaltbild mit dem Vibratormotor, einer Eingabeeinrichtung, einem Lagesensor und dem Kraftsensor.

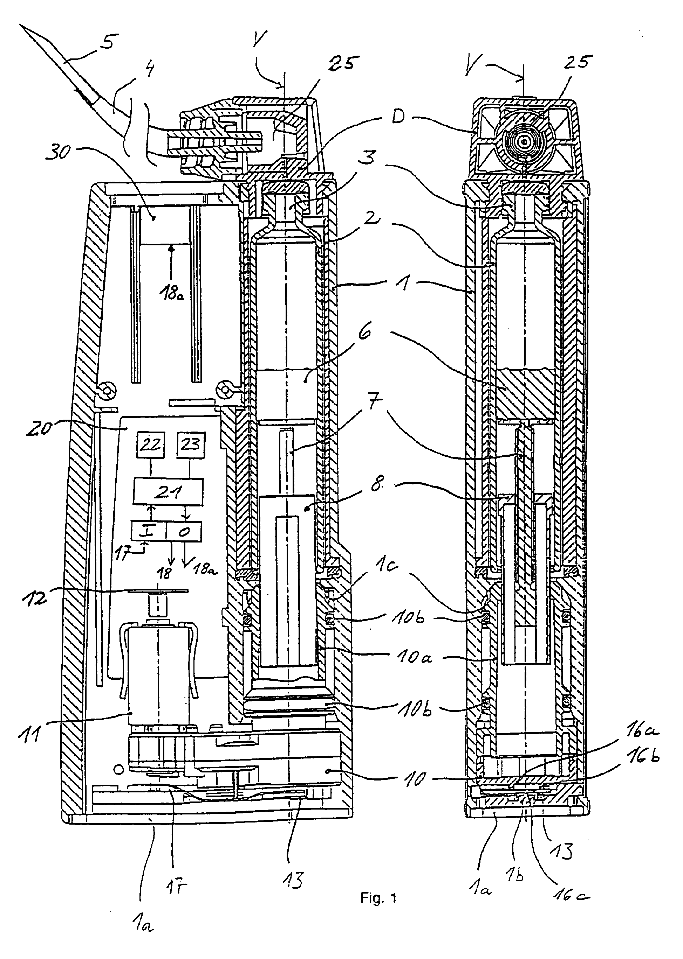

Fig. 1 zeigt eine tragbare Infusionspumpe für die Insulinbehandlung. Die Pumpe, insbesondere deren Antrieb, wird in der deutschen Patentanmeldung Nr. 197 17 107 beschrieben.

In einem Pumpengehäuse 1 ist ein Behältnis in Form einer Ampulle 2 aufgenommen. Die Ampulle 2 ist mit Insulin gefüllt. Ein Fördermittel in Form eines Kolben 6 ist in der Ampulle 2 in eine Vorschubrichtung auf einen Ampullenauslass 3 zu geradverschiebbar aufgenommen. Der Vorschub des Kolbens 6 wird durch Andruck eines als Gewindestange ausgebildeten Abtriebsglieds 7 auf eine rückwärtige Fläche des Kolbens 6 bewirkt. Das Abtriebsglied 7 ist Teil eines Spindeltriebs 8, der teleskopierbar zweistufig ausgebildet ist. Die Erfindung ist bezüglich des Kolbenantriebs jedoch hierauf nicht beschränkt.

Beim Vorschieben des Kolbens 6 entlang einer Verschiebeachse V wird Insulin über einen am Auslass 3 angeschlossenen Katheter 4 und eine am vorderen freien Ende des Katheters 4 befestigte Infusionsnadel 5 ausgeschüttet. Um in der Ampulle 2 einen definierten Basisdruck einzustellen, ist auf dem Strömungsweg des Insulins ein Ventil 25 angeordnet. Solch ein Ventil kann nicht nur zur Definition eines im Fluidführungssystem 2 - 5 stets herrschenden Basisdrucks verwendet werden, sondern auch zum Verhindern einer Selbstentleerung der Ampulle 2 unter dem Eigengewicht der Flüssigkeitssäule im Fluidführungssystem 2 - 5. Bevorzugterweise ist das Ventil 25 so ausgelegt, dass es solch eine unerwünschte Selbstentleerung unter den im praktischen Gebrauch der Infusionspumpe auftretenden Bedingungen sicher verhindert. Im Ausführungsbeispiel ist das Ventil 25 in einem abnehmbaren Gehäusedeckel D aufgenommen, mit dem ein Ampullenfach nach Einsetzen der Ampulle 2 verschlossen wird.

Der Antrieb des Abtriebsglieds 7 erfolgt mittels eines Antriebsmotors 11 über ein Untersetzungsgetriebe auf den Spindeltrieb 8 mit dem Abtriebsglied 7. In Bezug auf den Spindeltrieb und das Untersetzungsgetriebe wird auf die deutsche Patentanmeldung Nr. 197 17 107 verwiesen. Zumindest das Abtriebsglied 7 ist im Gehäuse 1 verdrehgesichert geradgeführt, so dass ein Drehantreiben von zwei vorgelagerten Antriebsgliedern des Spindeltriebs, die das Abtriebsglied 7 hülsenförmig umgeben, einen Vorschub der Gewindestange 7 bewirkt.

Der Spindeltrieb 8 ist zusammen mit dem Getriebe und dem Motor 11 auf einer Verschiebeplattform 10 gelagert, die ihrerseits als Ganzes im Gehäuse 1 verdrehgesichert geradverschiebbar in und gegen die Vorschubrichtung des Kolbens gelagert ist. Es wäre grundsätzlich auch möglich, den Motor 11 gehäusefest anzuordnen, ebenso wäre eine gehäusefeste Anordnung des Motors 11 zusammen mit dem Getriebe möglich. In diesem Fall müsste zwischen der gehäusefesten Antriebskomponente und der dann auf der Verschiebeplattform 10 gelagerten Eingangsstufe des Spindeltriebs 8 ein entsprechender Verschiebeeingriff, beispielsweise eine sich längs der Vorschubrichtung über eine entsprechende Länge erstreckende Stirnradverzahnung, vorgesehen sein.

Um etwaige Fehlfunktionen im Fluidführungssystem 2, 3, 4, 5 feststellen zu können, wird der Druck des Produktfluids, insbesondere der Fluiddruck in der Ampulle 2, überwacht. Für die Drucküberwachung wird eine vom Kolben 6 auf das Gehäuse 1 ausgeübte Reaktionskraft mittels eines Kraftsensors 13 gemessen und mit einem vorgegebenen Sollwert für die Reaktionskraft verglichen. Eine Kalibrierung des Sollwerts wird in der deuschen Patentanmeldung Nr. 19S 40 992 beschrieben.

Als Reaktionskraft wird mittels eines Kraftsensors 13 die Kraft gemessen, die vom Kolben 6 auf die Gewindestange 7 und über den Spindeltrieb auf die Verschiebeplattform 10 und infolge deren Verschiebbarkeit auf den Kraftsensor 13 ausgeübt wird. Zu diesem Zweck ist die Verschiebeplattform 10 an Gehäusewandungen im Gehäuse 1 schwimmend gelagert.

Hierzu ist die Verschiebeplattform 10 mit einer Plattformhülse 10a in einem diese Hülse 10a umgebenden Gehäusehülsenteil längsverschiebbar mittels elastischen Lagerelementen 10b in Form eines Paars von O-Ringen, beispielsweise Gummiringe, abgestützt. Die Plattformhülse 10a umgibt als Antriebshülsen ausgebildete Antriebsglieder des Spindeltriebs. Ein Kontakt zwischen der Verschiebeplattform 10 und dem Gehäuse 1 wird nur über diese O-Ringe 10b hergestellt. Eine Verschiebebewegung entlang der Verschiebeachse V findet zwischen der Verschiebeplattform 10 und dem Gehäuse 1 nur im Rahmen der elastischen Verformung der O-Ringe 10b statt. Die O-Ringe 10b selbst werden weder gegenüber dem Gehäuse 1 noch gegenüber der Verschiebeplattform 10 verschoben, sondern lediglich elastisch verformt. Durch diese Ausbildung der schwimmenden Lagerung werden die Reibungskräfte minimiert und die bei dem Verschieben des Kolbens 6 aufgebrachte Kraft weitestgehend unverfälscht auf den Kraftsensor 13 übertragen. Die O-Ringe 10b sind in umlaufenden Nuten der Plattformhülse 10a aufgenommen und auf diese Weise gegenüber diesen beiden sich gegenüberliegenden Flächen des Gehäuses 1 und der Plattformhülse 10a durch Formschluss einerseits und Kraftschluss andererseits fixiert. Sie könnten jedoch auch mit einer dieser beiden Flächen stoffschlüssig verbunden sein, und sie könnten auch bei entsprechender Montage zwischen den beiden gegeneinander sich bewegenden Flächen zusammengedrückt und derart nur kraftschlüssig gehalten werden. Allerdings sollte dennoch gewährleistet sein, dass mit Ausnahme der elastischen Verformungskräfte keine weiteren Reibungskräfte beim Verschieben der Verschiebeplattform 10 wirken.

Der Kraftsensor 13 ist zwischen einer rückwärtigen Stirnfläche der Verschiebeplattform 10 und einer dieser rückwärtigen Stirnfläche gegenüberliegenden Wandung des Gehäuses 1 angeordnet. Er ist ferner in der Flucht der Verschiebeachse V des Kolbens 6 angeordnet, so dass die längs der Verschiebeachse V des Kolbens 6 wirkende Reaktionskraft unmittelbar auf den Kraftsensor 13 wirkt. Die Reaktionskraft wird symmetrisch bezüglich der Verschiebeachse V eingeleitet. Ein Kippmoment durch die Reaktionskraft kann daher nicht entstehen.

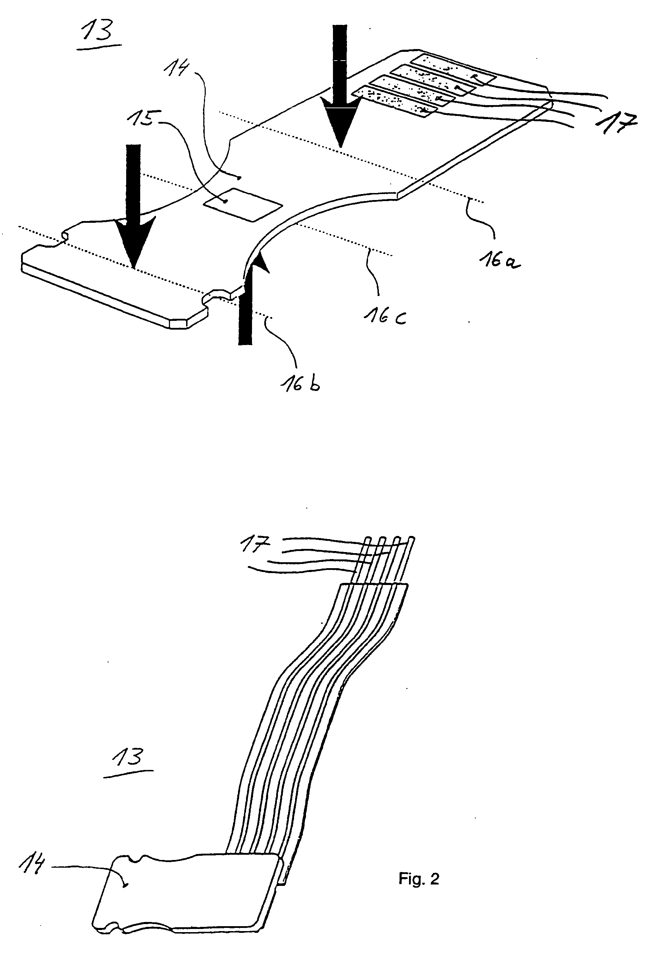

In Figur 2 ist der Kraftsensor 13 allein dargestellt. Er wird durch einen Biegebalken 14 gebildet, auf dem wenigstens an einer Balkenoberfläche ein Dünnfilm-Dehnungsmessstreifen 15 aufgebracht ist. Eine Messwertverstärkung könnte durch Aufbringen auf beiden sich gegenüberliegenden Balkenoberflächen erzielt werden. Vier Leitungen einer Brückenschaltung sind mit 17 bezeichnet. In Ausbildung des Biegebalkens sind an der einen Balkenoberfläche voneinander parallel beabstandet zwei Stege 16a und 16b (Figur 1) als plattformseitige, linienförmige Auflagen angedeutet, zwischen denen der Balken 14 mit dem Dehnungsmessstreifen 15 durch die vom Kolben 6 ausgeübte Reaktionskraft gebogen wird. Zur exakten Definition des Ortes der Einleitung der Reaktionskraft ragt von einer Bodenplatte 1b von der den beiden Stegen 16a und 16b gegenüberliegenden Unterseite des Gehäuses 1 exakt in der Mitte zwischen diesen beiden Stegen 16a und 16b ein weiterer Steg 16c ab (Figur 1), dessen linienförmige Auflage in Fig. 2 angedeutet ist. Die linienförmige Auflage des dritten Stegs 16c liegt in der Flucht der Verschiebeachse V und parallel zu den Stegen 16a und 16b. Die drei Stege 16a, 16b und 16c sind im Auflagebereich im Querschnitt rund, so dass die Reaktionskraft entlang der Stege 16a und 16b möglichst exakt linienförmig eingeleitet wird und auch die Auflagerlast linienförmig bei dem Steg 16c auf den Biegebalken 14 wirkt. Andere Querschnittsformen, die dies bewirken oder annähern, sind auch geeignet. Andere Sensoren, beispielsweise piezo-resistive Sensoren, wären statt Dehnungsmessstreifen im Rahmen eines statischen Messverfahrens ebenfalls verwendbar.

Vom Kraftsensor 13 wird ein die aufgeprägte Reaktionskraft repräsentierender, vorzugsweise zur Reaktionskraft proportionaler Messwert, vorzugsweise in Form eines elektrischen Messwertsignals. über eine Leitung 17 auf die Steuerung 20 für den Motor 11 ausgegeben. Der die aktuell gemessene Reaktionskraft repräsentierende Messwert liegt permanent an einem Eingang I der Steuerung 20. Über einen Ausgang O und eine entsprechende Steuerungsleitung bzw. einen Steuerungsbus 18 ist die Steuerung 20 mit dem Motor 11 verbunden. Der Motor 11 wird positionsgesteuert.

In dem Gehäuse 1 ist ein Vibratormotor 30 angeordnet. Der Vibratormotor 30 is: starr an dem Gehäuse 1 befestigt. Im Ausführungsbeispiel ist er in einem Verschlussstopfen des Gehäuses 1 befestigt, vorzugsweise steif, damit die Vibrationen auf das Gehäuse 1 übertragen werden. Der Vibratormotor 30 ist mit dem Ausgang O der Steuerung 20 über eine Leitung 18a verbunden, d.h. er wird von der Steuerung 20 über die Leitung 18a angesteuert.

Wird mit Hilfe des Kraftsensors 13 von der Steuerung 20 durch Vergleich der gemessenen Reaktionskraft mit einem Höchstwert eine Occlusion festgestellt, so wird der Vibratormotor 30 von der Steuerung 20 über die Leitung 18a angesteuert und gibt ein die Occlusion anzeigendes, charakteristisches Vibrationssignal über das Gehäuse 1 ab.

Der Antriebsmotor 11 ist ein Schrittmotor mit physikalisch vorgegebener Start-Stop-Frequenz. Hierbei handelt es sich um eine Frequenz und dementsprechende Motordrehzahl, bei deren Überschreiten das Motordrehmoment abnimmt, wodurch der Motor 11 stillgesetzt wird, falls er in diesem Zustand einen vergleichsweise geringen Widerstand erfährt. Nach dem Stillsetzen fährt er nicht mehr selbsttätig an, sondern schwingt nur noch hin und her, bis er ganz abgestellt wird. Durch einen Steuerungsbefehl der Steuerung 20 wird er anschließend wieder angefahren.

Die Position des Motors 11 wird mittels eines auf der Motorrotorachse befestigen Flügelrads 12 und eines damit zusammenwirkenden optischen Sensors, für den das Flügelrad 12 als Lichtschrankenunterbrecher dient, überwacht. Die Steuerung 20 stellt den Motor 11 ab, falls ein Steuerungsimpuls nicht zu einer Motordrehung führt. Der Steuerung 20 ist die Motorposition zumindest in Form der Anzahl der zu jedem Zeitpunkt aus einer Referenzposition heraus zurückgelegten Umdrehungen bekannt. Gegebenenfalls kann die Motorposition auch mittels eines an den optischen Sensor angeschlossenen Zählwerks ermittelt werden, das die Zahl der Unterbrechungen durch die Flügel des Flügelrads 12 hochzählt. Damit kennt die Steuerung auch die Position des Abtriebsglieds 7 relativ zur Verschiebeplattform 10 und letztlich auch die Position des Kolbens 6 in der Ampulle 2.

Die Steuerung 20 umfasst einen Mikroprozessor 21 mit zwei nicht flüchtigen Speichern 22 und 23. Im Speicher 22 ist ein Standard-Sollwertverlauf S für die Reaktionskraft gespeichert. Der weitere Speicher 23 wird bei einem Primen der Infusionspumpe beschrieben. Die Steuerung 20 ist über eine Schnittstelle I/0 mit dem Motor 11, dem Kraftsensor 13 und weiteren Komponenten, insbesondere einer Energiequelle, verbunden. Die Verbindung zum Kraftsensor 13 ist mit dem Bezugszeichen 17 und die zum Motor 11 mit dem Bezugszeichen 18 angedeutet.

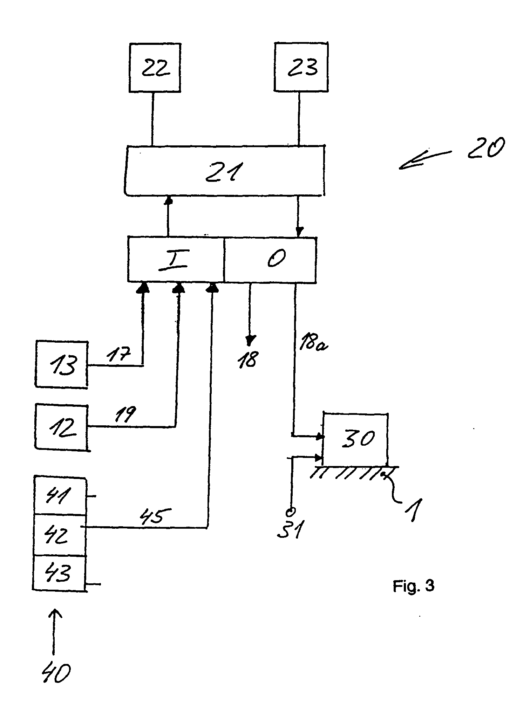

Figur 3 zeigt die Einbettung des Vibratormotors 30 in die Drucküberwachung und Überwachung des Antriebsmotors 11. Ferner ist der Vibratormotor 30 über die Steuerung 20 mit einer Eingabeeinrichtung 40 verbunden. Die Drucküberwachung erfolgt mittels des Kraftsensors 13, dessen Messwertsignal über die Leitung 17 zur Steuerung 20 gelangt. Falls die Steuerung 20 durch Vergleich der von dem Kraftsensor 13 gemessenen Kraft mit einem Maximalwert eine Occlusion feststellt, schaltet sie über die Leitung 18a den Vibratormotor 30 ein. Der Benutzer kann den Vibratormotor über eine Leitung 31 mittels der Eingabeeinrichtung 40 mannuell abschalten. Im Falle einer mit Hilfe des Sensors 12 festgestellten Fehlfunktion des Motors 11 wird der Vibratormotor 30 ebenfalls von der Steuerung 20 eingeschaltet. Insbesondere schaltet sie den Vibratormotor 30 ein, wenn sie wie vorstehend beschrieben den Motor 11 abschaltet. Die Antriebsüberwachung erfolgt mittels des Flügelrads und des damit zusammenwirkenden optischen Sensors, die zusammen in Figur 3 mit den Bezugszeichen 12 bezeichnet sind.

Die Eingabeeinrichtung 40 wird durch eine Tastatur oder ein Touchscreen gebildet. Mit den Bezugszeichen 41, 42 und 43 sind stellvertretend für weitere die einzelnen Eingabemittel der Eingabeeinrichtung 40 bezeichnet, die entweder durch Tasten oder Tastenfelder gebildet werden. Jedes der Eingabemittel der Eingabeeinrichtung 40 ist mit dem Eingang I der Steuerung 20 über je eine Leitung 45 elektrisch verbunden. Stellvertretend für die mehreren Leitungen 45 ist nur eine Leitung bis zur Steuerung 20 durchgezeichnet. Die Steuerung 20 gibt über die Verbindungsleitung 18a in Abhängigkeit von dem aktuell gerade betätigten Eingabemittel der Eingabeeinrichtung 40 ein individuelles Steuersignal ab, und der Vibratormotor 30 erzeugt in Abhängigkeit von diesem Steuersignal ein für jedes der Eingabemittel charakteristisches Vibrationssignal.

Beim Primen wird aus einem Ausgangszustand, in dem die Ampulle 2 in das Gehäuse 1 eingesetzt und ihre Verschlussmembran am Auslass 3 durch eine Verbindungsnadel durchstochen wurde, die Gewindestange 7 auf den Kolben 6 zu verfahren. Bis zum Anstoßen an den Kolben 6 fährt der Motor 11 im Schnellgang deutlich oberhalb der Start-Stop-Frequenz.

Im Schnellgang wird er mit einer Pulsfrequenz betrieben, die vorzugsweise wenigstens doppelt so groß wie seine Start-Stop Frequenz ist. Sobald die Gewindestange 7 mit Ihrem Stempel an den Kolben 6 anstößt, stellt sich der Motor 11 selbsttätig ab, da sein Drehmoment oberhalb der Start-Stop-Frequenz nicht ausreicht, die Gewindestange 7 und den Kolben 6 anzutreiben. In der Steuerung 20 wird die Stopposition des Motors 11 registriert und als Nullposition für die Produktausschüttung gespeichert. Falls für den verwendeten Ampullentyp in der Steuerung 20 ein geeigneter Referenzwert für eine bestimmte Kolbenposition gespeichert ist, kann die Steuerung 20 durch Vergleich mit diesem Referenzwert aus der beim Anfahren an den Kolben festgestellten tatsächlichen Kolbenposition ermitteln, ob es sich bei der Ampulle 2 um eine volle oder beispielsweise halbvolle Ampulle handelt.

Im Ausführungsbeispiel wird der als Schrittmotor mit einstellbarer Start-Stop-Frequenz ausgebildete Motor 11 nach dem vorbeschriebenen Stillsetzen automatisch wieder angefahren, so dass nun der Kolben 6 in der Ampulle 2 vorgeschoben wird. Im Zuge des Primens wird der Kolben 6 in der Ampulle 2 auf den Auslass 3 zu so lange verschoben, bis Insulin an der Austrittsstelle der Infusionsnadel 5 austritt. Mit dem Wiederanfahren des Motors 11 wird der Vibratormotor 30 von der Steuerung 20 angesteuert und dadurch eingeschaltet. Durch die Vibration des Vibratormotors 30 werden in dem Fluidführungssystem der Vorrichtung festsitzende Gasbläschen gelöst, so dass mit Abschluss des Primens eine sichere Entlüftung des Fluidführungssystems gewährleistet ist. Sobald der Fiuidaustritt sicher festgestellt ist, wird das Abtriebsglied 7 und damit auch der Kolben 6 in der gerade erreichten Verschiebeposition entlang der gemeinsamen Verschiebeachse V stillgesetzt. Das Stillsetzen kann manuell erfolgen, wenn der Austritt vom Verwender beobachtet wird, oder auch automatisch durch die Steuerung 20. Mit dem Stillsetzen, insbesondere durch Abschalten des Motors 11, wird gleichzeitig auch der Vibratormotor 30 von der Steuerung 20 über die Leitung 18a stillgesetzt bzw. abgeschaltet. Der Vibratormotor 30 ist dann wieder bereit für den Empfang von Steuersignalen, beispielsweise im Falle einer festgestellten Occlusion oder zur Bestätigung von Eingaben mittels der Eingabeeinrichtung 40.

### Bezugszeichenliste

- 1: Gehäuse
- 2: Behältnis
- 3: Auslass
- 4: Katheter
- 5: Nadel
- 6: Fördermittel, Kolben
- 7: Abtriebsglied
- 8: Spindeltrieb
- 9: -----
- 10: Verschiebeplattform
- 10a: Hülse
- 10b: Lagerelement, O-Ring
- 11: Antriebsmotor, Schrittmotor
- 12: Sensor, Flügelrad
- 13: Sensor, Kraftsensor
- 14: Biegebalken
- 15: Dehnungssensor
- 16: Positionier- und Befestigungseinrichtung
- 16a: Steg
- 16b: Steg
- 16c: Steg
- 17: Leitung
- 18: Steuerungsleitung
- 18a: Steuerungsleitung
- 19: Leitung
- 20: Steuerung
- 21: Mikroprozessor
- 22: Speicher
- 23: Speicher
- 24: ------
- 25: Ventil
- 26-29: ------
- 30: Vibratormotor
- 31: Steuerungsleitung
- 40: Eingabeeinrichtung
- 41-43: Eingabemittel

- D: Gehäusedeckel
- V: Verschiebeachse

## Patentansprüche

1. Vorrichtung zur dosierten Verabreichung eines injizierbaren Produkts umfassend:
a) ein Gehäuse (1),
b) ein von dem Gehäuse (1) aufgenommenes Behältnis (2) für das Produkt,
c) ein Fördermittel (6) zur Förderung des Produkts aus dem Behältnis (2),
d) eine Antriebseinrichtung (7, 8, 11) für das Fördermittel (6),
e) eine Einrichtung (13, 20) zur Feststellung einer Fehlfunktion der Vorrichtung umfassend eine Steuerung (20) und einen Kraftsensor (13) zur Überwachung eines Drucks des Produkts,
**dadurch gekennzeichnet, dass** der Kraftsensor (13) eine vom Fördermittel (6) auf das Gehäuse (1) ausgeübte Reaktionskraft misst, dass das Gehäuse (1) eine Verschiebeplattform (10) umfasst, wobei der Kontakt zwischen Gehäuse (1) und Verschiebeplattform (10) über O-Ringe (10b) hergestellt wird, dass die Reaktionskraft via der Verschiebeplattform (10) auf den Kraftsensor (13) übertragen wird, und dass ein vom Gehäuse (1) aufgenommener Vibratormotor (30) bei Feststellung einer Fehlfunktion durch die Einrichtung (13, 20) zur Feststellung einer Fehlfunktion der Vorrichtung, welche auf einer Messung des Kraftsensors (13) basiert, so angesteuert wird, dass er ein vibratorisches Alarmsignal erzeugt.

2. Vorrichtung gemäss Anspruch 1, **dadurch gekennzeichnet, dass** der Sensor (13) den Druck innerhalb des Behältnisses (2) für das Produkt misst.

3. Vorrichtung gemäss Anspruch 1 oder 2, **dadurch gekennzeichnet, dass** die Steuerung (20) einen Messwert des Sensors (13) mit einem gespeicherten Sollwert für den Messwert vergleicht und in Abhängigkeit des Vergleichs den Vibratormotor (30) ansteuert.

4. Vorrichtung gemäss einem der vorherigen Ansprüche, **dadurch gekennzeichnet, dass** das Fördermittel (6) einen Kolben (6), ein Abtriebsglied (7) und einen Spindeltrieb (8) umfasst und der Kraftsensor (13) die durch den Kolben (6) über das Abtriebsglied (7) und den Spindeltrieb (8) auf das Gehäuse (1) ausgeübte Reaktionskraft misst.

5. Vorrichtung gemäss einem der vorherigen Ansprüche **dadurch gekennzeichnet, dass** der Kraftsensor (13) einen Biegebalken (14) und einen Dünnfilm-Dehnungsmessstreifen (15) umfasst.

## Claims

1. Device for administering an injectable product in doses, said device comprising:
a) a casing (1),
b) a container (2) for the product, said container (2) being accommodated by the casing (1),
c) a delivering appliance (6) for delivering the product from the container (2),
d) a drive means (7, 8, 11) for the delivering appliance (6),
e) a means (13, 20) for determining a malfunction of the device comprising a control system (20) and a force sensor (13) for monitoring a pressure of the product,
**characterized in that** the force sensor (13) measures a reaction force exerted by the delivering appliance (6) on the casing (1), that the casing (1) comprises a sliding platform (10), wherein the contact between the casing (1) and the sliding platform (10) is established via O-rings (10b), that the reaction force is exerted via the sliding platform (10) onto the force sensor (13), and that a vibrator motor (30), said vibrator motor (30) being accommodated by the casing (1), is triggered by the means (13, 20) for determining a malfunction of the device upon determination of a malfunction, which is based on a measurement of the force sensor (13), such that it generates a vibrating alarm signal.

2. Device according to claim 1, **characterized in that** the sensor (13) measures the pressure in the container (2) for the product.

3. Device according to claim 1 or 2, **characterized in that** the control system (20) compares a reading of the sensor (13) with a stored index value for the reading and triggers the vibrator motor (30) depending on the comparison.

4. Device according to one of the preceding claims, **characterized in that** the delivering appliance (6) comprises a piston (6), a driven member (7) and a spindle drive (8) and the force sensor (13) measures the reaction force exerted by the piston (6) via the driven member (7) and the spindle drive (8) on the casing (1).

5. Device according to one of the preceding claims, **characterized in that** the force sensor (13) comprises a bending beam (14) and a thin-film strain measuring strip (15).

## Revendications

1. Dispositif pour l'administration dosée d'un produit injectable comprenant :
a) un boîtier (1),
b) un récipient (2) pour le produit, monté dans le boîtier (1),
c) un moyen d'alimentation (6) pour faire sortir le produit du récipient (2),
d) un dispositif d'entraînement (7, 8, 11) pour le moyen d'alimentation (6),
e) un dispositif (13, 20) pour la détermination d'une fonction de panne du dispositif comprenant une dispersion (20) et un capteur de force (13) pour contrôler la pression du produit,
**caractérisé en ce que** le capteur de force (13) mesure une force de réaction exercée par le moyen d'alimentation (6) sur le boîtier (1), **en ce que** le boîtier (1) comprend une plate-forme de déplacement (10), le contact entre le boîtier (1) et la plate-forme de déplacement (10) étant établi par l'intermédiaire de joints toriques (10b), **en ce que** la force de réaction est transférée via la plate-forme de déplacement (10) sur le capteur de force (13), et **en ce qu'**un moteur de vibrateur (30) reçu par le boîtier (1), dans le cadre de la détermination d'une fonction d'erreur par le dispositif (13, 20) pour la détermination d'une fonction erreur du dispositif, qui repose sur une mesure du capteur de force (13), est commandé de telle sorte qu'il produit un signal d'alarme vibrant.

2. Dispositif selon la revendication 1, **caractérisé en ce que** le capteur (13) mesure la pression à l'intérieur du récipient (2) pour le produit.

3. Dispositif selon la revendication 1 ou 2, **caractérisé en ce que** la commande (20) d'une valeur de mesure du capteur (13) effectue la comparaison avec une valeur nominale mémorisée pour la valeur de mesure et, en fonction de la comparaison, pilote le moteur de vibrateur (30).

4. Dispositif selon l'une des revendications précédentes, **caractérisé en ce que** le moyen d'alimentation (6) comprend un piston (6), un élément d'entraînement (7) et un entraînement de broche (8) et le capteur de force (13) mesure la force de réaction exercée par le piston (6) par l'intermédiaire de l'élément de sortie (7) et l'entraînement de broche (8) sur le boîtier (1).

5. Dispositif selon l'une des revendications précédentes, **caractérisé en ce que** le capteur de force (13) comprend un axe de flexion (14) et une bande de mesure de dilatation à film mince (15).
